# EUROPÄISCHE PATENTANMELDUNG

(11) **EP 1 449 838 A1**
(43) Veröffentlichungstag der Anmeldung: **25.08.2004**
(21) Anmeldenummer: 04007390.0
(22) Anmeldetag: 09.05.2001
(51) Int. Cl.: C07D 307/83

(54) **Verfahren zur Herstellung von 2-Coumaron und substituierten 2-Coumaronen**

(30) Priorität: 06.06.2000 AT 983002000
(62) Teilanmeldung aus: 01940421.9
(71) Anmelder: DSM Fine Chemicals Austria Nfg GmbH & Co KG, 4021 Linz (AT)
(72) Erfinder: Stanek, Michael, 4020 Linz (AT); Hildebrand, Peter, 4020 Linz (AT); Zimmermann, Curt, 4312 Ried in der Riedmark (AT); Castelijns, Marianne, 6176 JB Spaubeek (NL)
(74) Vertreter: Lindinger, Ingrid

(57) **Zusammenfassung**

Verfahren zur Herstellung von 2-Coumaron oder substituierten 2-Coumaronen, bei welchem Cyclohexanon oder substituierte Cyclohexanone mit einem carboxylhältigen Acylierungsmittel
a) zu 2-(2-Oxo-cyclohexyl)-2-hydroxy-essigsäurealkylester oder substituierten 2-(2-Oxo-cyclohexyl)-2-hydroxy-essigsäurealkylestern umgesetzt werden, die
   a₂) durch azeotrope Destillation unter basischen Bedingungen zu einem Gemisch aus 2-Oxocyclohexyliden-essigsäurealkylester und dem Enol-Lacton der 2-Oxo-cyclohexyliden-essigsäure oder einem Gemisch aus substituiertem 2-Oxocyclohexyliden-essigsäurealkylester und dem Enol-Lacton der substituierten 2-Oxocyclohexyliden-essigsäure dehydratisiert wird, das anschließend wiederum durch katalytische Gasphasen-Dehydrierung in 2-Coumaron oder substituierte 2-Coumarone überführt wird, oder
b) direkt unter basischen Bedingungen zu einem Gemisch aus 2-Oxocyclohexyliden-essigsäurealkylester und dem Enol-Lacton der 2-Oxocyclohexylidenessigsäure oder einem Gemisch aus substituiertem 2-Oxocyclohexyliden-essigsäurealkylester und dem Enol-Lacton der substituierten 2-Oxo-cyclohexyliden-essigsäure umgesetzt wird, das anschließend wiederum durch katalytische Gasphasen-Dehydrierung in 2-Coumaron oder substituierte 2-Coumarone überführt wird.

## Beschreibung

2-Coumaron stellt einen wichtigen Rohstoff zur Synthese von Agrochemikalien dar. Aus der Literatur sind bereits verschiedene Verfahren zur Herstellung von 2-Coumaron, das auch als 3H-2-Benzofuran-2-on oder 2-Coumaranon bezeichnet wird, bekannt. Sie beruhen teils auf mehrstufiger Synthese und teils auf katalytischer Dehydrierung in der Gasphase.
R. W. Layer et al. (US 3 862 133) beschreiben die Synthese zur Herstellung von γ-Lactonen der o-Hydroxyphenylessigsäure, wobei der Aromat Substituenten trägt. Im Falle der Verwendung von Phenol und Glyoxal werden nur sehr geringe Ausbeuten an 2-Coumaron erhalten, da auf Grund der fehlenden Substituenten am Aromaten mehrere reaktive Positionen zu vielen Nebenprodukten führen.
Ebenfalls substituierte 3H-2-Benzofuran-2-one werden laut Beschreibung von Pfitzer Inc. (GB 1 337 507) ausgehend von substituierter 2-Methoxy-phenylessigsäure hergestellt. Die Synthese der 2-Methoxy-phenylessigsäure, anschließende Etherspaltung und Cyclisierung zu 2-Coumaron stellt kein kostengünstiges Verfahren dar. Eine andere Methode zur Herstellung von 5-Chloro-3-H-benzofuran-2-on beschreiben J. C. Vallejos et al (FR 2 686 880), wobei von p-Chlorphenol und Glyoxylsäure in Anwesenheit von Phosphinsäure und katalytischen Mengen an Jod oder Salzsäure ausgegangen wird.
Andere Methoden zur Herstellung von 2-Coumaron sind zwar in der Literatur erwähnt, haben aber nie den Stellenwert einer industriellen Synthese route erreicht: T. Fukagawa et al. berichten über die intramolekulare Acyloxylation der Phenylessigsäure (J. Org. Chem., 1982, 47, 2491); E. Baciocchi et al. verwenden trans-2,3-Dichloro-2,3-dihydrobenzofuran als Edukt (J. Org. Chem., 1979, 44, 32) und H. E. Holmquist beschreibt eine Synthese ausgehend von o-Kresol und Kohlenmonoxid.
Unlängst beschrieben J. C. Vallejos et al. (US 5 616 733) ein Verfahren zur Herstellung von 2-Coumaron durch katalytische Dampfphasen-Dehydrierung des Kondensationsproduktes aus Cyclohexanon und wäßriger Glyoxylsäure, das vorerst in konzentrierter Essigsäure gelöst wird. Der größte Nachteile dieses Verfahrens liegt neben der raschen Katalysatordeaktivierung darin, dass nur sehr geringe Ausbeuten an 2-Coumaron erhalten werden.

In einer weiteren Literaturstelle beschreiben J. C. Vallejos et al. (US 5 773 632), dass durch Synthese des Enol-Lactons des Kondensationsproduktes aus Cyclohexanon und wässriger Glyoxylsäure und anschließender katalytischer Gasphasen-Dehydrierung die Ausbeute an 2-Coumaron gesteigert werden kann. Die Herstellung des Kondensationsproduktes aus Cyclohexanon und 50 %iger wässriger Glyoxylsäure bedarf nachteilig der Verwendung von zusätzlichem Wasser und Salzsäure, die nach erfolgter Reaktion neben dem Überschuss an Cyclohexanon abdestilliert werden müssen. In einem weiteren Syntheseschritt erfolgt dann die Enol-Lactonisierung des Reaktionsgemisches, bestehend aus 2-Oxo-cyclohexyliden-essigsäure (cis und trans) und dem Enol-Lacton der 2-Oxo-cyclohexyliden-essigsäure. Das hauptsächlich gebildete Enol-Lacton der 2-Oxo-cyclohexyliden-essigsäure wird nach Vakuumdestillation der Dampfphasen-Dehydrierung ausgesetzt.

Unerwarteterweise konnte ein verbessertes Verfahren zur Herstellung von 2-Coumaron und substituierten 2-Coumaronen durch katalytische Gasphasen-Dehydrierung der Reaktionsprodukte aus Cyclohexanon oder substituierten Cyclohexanonen und einem carboxylhältigen Acylierungsmittel gefunden werden, bei welchem nicht nur die Reaktionsprodukte aus Cyclohexanon oder substituierten Cyclohexanonen und dem carboxylhältigen Acylierungsmittel, sondern auch 2-Coumaron und die substituierten 2-Coumarone in hohen Ausbeuten erhalten werden.

Gegenstand der Erfindung ist daher ein Verfahren zur Herstellung von 2-Coumaron oder substituierten 2-Coumaronen, das dadurch gekennzeichnet ist, dass Cyclohexanon oder substituierte Cyclohexanone mit dem carboxylhältigen Acylierungsmittel
a) zu 2-(2-Oxo-cyclohexyl)-2-hydroxy-essigsäureester oder substituierten 2-(2-Oxocyclohexyl)-2-hydroxy-essigsäureester umgesetzt werden, die
   a₁) entweder direkt durch katalytische Gasphasen-Dehydrierung in 2-Coumaron oder substituierte 2-Coumarone überführt werden, oder
   a₂) durch azeotrope Destillation unter basischen Bedingungen, oder unter Verwendung einer starken Säure, oder einem stark sauren lonentäuscher zu einem Gemisch aus 2-Oxocyclohexyliden-essigsäurealkylester und dem Enol-Lacton der 2-Oxocyclohexyliden-essigsäure oder einem Gemisch aus substituiertem 2-Oxocyclohexyliden-essigsäurealkylester und dem Enol-Lacton der substituierten 2-Oxocyclohexyliden-essigsäure dehydratisiert wird, das anschließend wiederum durch katalytische Gasphasen-Dehydrierung in 2-Coumaron oder substituierte 2-Coumarone überführt wird, oder
b) direkt unter sauren oder basischen Bedingungen zu einem Gemisch aus 2-Oxocyclohexyliden-essigsäurealkylester und dem Enol-Lacton der 2-Oxocyclohexyliden-essigsäure oder einem Gemisch aus substituiertem 2-Oxocyclohexyliden-essigsäurealkylester und dem Enol-Lacton der substituierten 2-Oxo-cyclohexyliden-essigsäure umgesetzt wird, das anschließend wiederum durch katalytische Gasphasen-Dehydrierung in 2-Coumaron oder substituierte 2-Coumarone überführt wird.

Bei dem erfindungsgemäßen Verfahren wird von Cyclohexarion oder substituierten Cyclohexanonen der Formel (I) 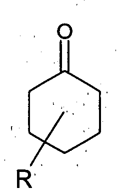 in der R H oder ein C₁-C₃-Alkyl-oder Alkoxyrest ist,
und einem carboxylhältigen Acylierungsmittel der allgemeinen Formeln (IIa) oder (IIb) 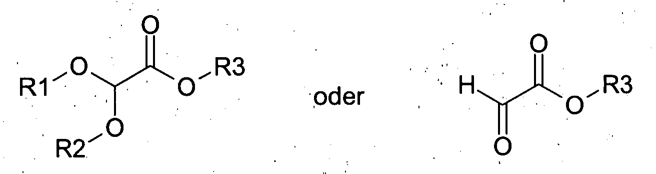 ausgegangen, wobei im Falle der Herstellung von 2-Coumaron R1, R2 und R3 unabhängig voneinander ein Methyl-, Ethyl- oder Propyl-Rest, oder R1 und R2 ein Wasserstoffatom und R3 eine Methyl-, Ethyl- oder Propyl-gruppe sein können und im Falle der Herstellung von substituierten Coumaronen R3 auch ein Wasserstoffatom sein kann.
Beispiele für Verbindungen der Formeln IIa und IIb sind Glyoxylsäure, Glyoxylsäuremethylester, Glyoxylsäureethylester, Glyoxylsäuremethylester-methylhemiacetal, Glyoxylsäuremethylester-dimethylacetal, Glyoxylsäureethylester-ethylhemiacetal.

Als carboxylhältiges Acylierungsmittel können auch Gemische von Verbindungen der Formel IIa und/oder der Formel IIb verwendet werden.

Der Reaktionsverlauf ist aus dem nachfolgenden Formelschema ersichtlich - R steht für Methyl, Ethyl, Propyl, Methoxy, Ethoxy oder H:

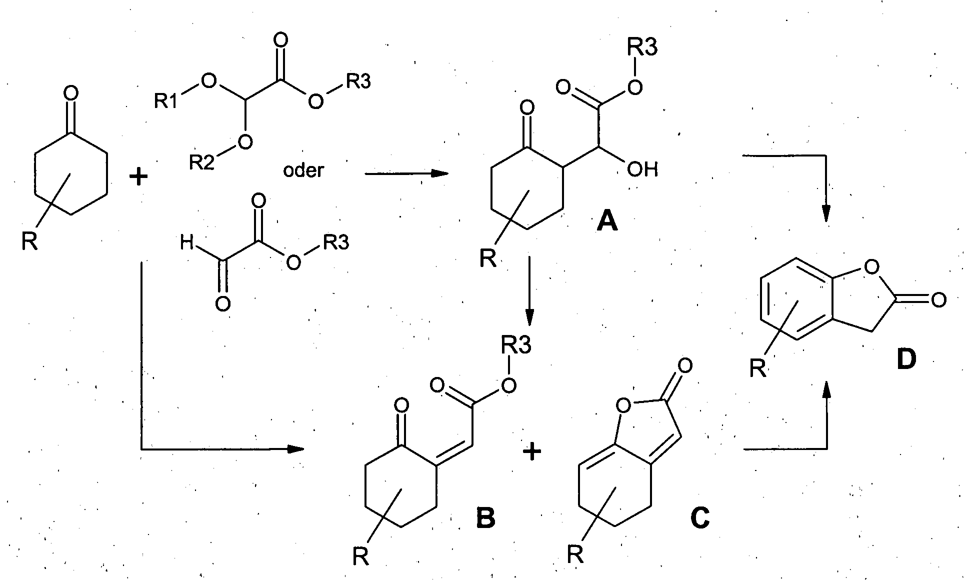

Das Reaktionsprodukt A, der 2-(2-Oxo-cyclohexyl)-2-hydroxy-essigsäurealkyl-ester oder substituierte 2-(2-Oxo-cyclohexyl)-2-hydroxy-essigsäurealkyl-ester, wird erfindungsgemäß nahezu quantitativ durch Umsetzung von Cyclohexanon oder substituierten Cyclohexanonen und dem carboxylhältigen Acylierungsmittel unter inerter Atmosphäre erhalten, wobei vorzugsweise das carboxylhältige Acylierungsmittel zu Cyclohexanon oder dem substituierten Cyclohexanon langsam zudosiert und der dabei anfallende Alkohol (Methanol, Ethanol, Propanol) über Kopf aus dem Reaktionsgemisch beseitigt wird. Das molare Verhältnis zwischen Cyclohexanon oder dem substituierten Cyclohexanon und dem carboxylhältigen Acylierungsmittel wird zwischen 100:1 und 0,5 :1 gewählt. Bevorzugt beträgt das molare Verhältnis zwischen Cyclohexanon oder dem substituierten Cyclohexanon und dem carboxylhältigen Acylierungsmittel zwischen 20:1 und 1:1, besonders bevorzugt zwischen 10: 1 und 1,5:1. Die Temperatur der Reaktionsmischung beträgt 50 bis 190 °C, bevorzugt 80 bis 150 °C und besonders bevorzugt 95 bis 145 °C. Das Ende der Reaktion wird durch eine geeignete Inprozeß-kontrolle auf das Abreagieren des carboxylhältigen Acylierungsmittels festgestellt. Nach erfolgter Reaktion wird überschüssiges Cyclohexanon oder substituiertes Cyclohexanon unter vermindertem Druck aus dem Reaktionsgemisch entfernt. Das Reaktionsprodukt A kann gegebenenfalls unter Vakuum destilliert werden, wobei bevorzugt bei einem Vakuum < 0.5 mbar destilliert wird.
Das Reaktionsprodukt 2-(2-Oxo-cyclohexyl)-2-hydroxy-essigsäurealkylester (= Reaktionsprodukt A) ist zwar aus der Literatur bekannt und wurde bisher entweder durch enzymatische Reduktion des entsprechenden 2-Oxo-Säureesters (A. Schummer et al, Tetrahedron, Vol., 47, 1991, Seite 9019; S. Tsuboi et al, J. Org. Chem., 1987, 52, 1359; S. Tsuboi et al, Tetrahedron Letters, Vol. 27, 1986, Seite 1915), oder durch Reaktion von Cyclohexanon, sekundärem Amin und Glyoxylsäure in einer Art Mannich Reaktion und anschließender Veresterung erhalten (J. Schreiber et al, Bull. Soc.

Chim. Fr., 1973, 2, Seite 625). Die Herstellung in einem einzigen Syntheseschritt aus billigen Rohstoffen war bisher jedoch nicht bekannt und ist deshalb ein weiterer Gegenstand der vorliegenden Erfindung.

Bei dem erfindungsgemäßen Verfahren wird in Schritt a2) das Reaktionsprodukt A nach Zugabe eines organischen Lösungsmittels und gegebenenfalls einer Säure oder sauren Ionentauschers durch Wasserauskreisen dehydratisiert, wobei ein Gemisch aus 2-Oxocyclohexyliden-essigsäurealkylester (Reaktionsprodukt B, cis- oder trans-Form) und dem Enol-Lacton der 2-Oxo-cyclohexyliden-essigsäure (Reaktionsprodukt C) oder einem Gemisch aus substituiertem 2-Oxocyclohexyliden-essigsäurealkylester (Reaktionsprodukt B, cis- oder trans-Form) und dem substituierten Enol-Lacton der 2-Oxo-cyclohexyliden-essigsäure (Reaktionsprodukt C) erhalten wird. Je nach Reaktionsführung, aber auch durch Zudosierung von Wasser, kann das Verhältnis zwischen B und C gesteuert werden. Die Reaktionstemperatur beträgt mehr als 50 °C, besonders bevorzugt zwischen 80 und 160 °C. Als Säuren eignen sich insbesondere diejenigen, die in einem organischen Medium löslich sind, wobei bevorzugt p-Toluolsulfonsäure, Methansulfonsäure, konzentrierte Schwefelsäure, etc., eingesetzt werden, jedoch besonders bevorzugt wird ein stark saurer lonentauscher verwendet. Als organische Lösungsmittel eignen sich bevorzugt aliphatische oder aromatische, gegebenenfalls halogenierte Kohlenwasserstoffe wie etwa Benzol, Toluol, Xylole und Tetrachlorkohlenstoff, aber auch andere organische Lösungsmittel, die laut klassischer Literatur zum Auskreisen von Wasser dienen.

Die nichtsubstituierten Reaktionsprodukte B und C sind in der Literatur durch Synthese über eine andere, aber wirtschaftlich nicht effiziente Herstellungsroute, als die erfindungsgemäße Variante, bekannt: A. Mondon et al, Chem. Ber., 96, 826, 1963; K. W. Rosenmund et al, Arch. Pharmarz. Ber. dtsch. pharmaz. Ges, 287, 441; A. W. Noltes et al, Rec. Trav. Chim., 80, 1334, 1961; Y. Arbuzov et al, Zhur. Obshch. Khim., 32, 3676, 1962, M. N. Kolosov et al, Zhur. Obshch. Khim., 32, 2983, 1962; F. Bonadies et al, Gazz. Chim. Ital., 113, 421; 1983.

Das Gemisch der Reaktionsprodukte B und C kann erfindungsgemäß aber auch in einem einzigen Syntheseschritt (Schritt b) durch Umsetzung von Cyclohexanon oder subsituiertem Cyclohexanon und dem carboxylhältigen Acylierungsmittel unter sauren oder basischen, vorzugsweise jedoch unter sauren Bedingungen, und unter Schutzgasatmosphäre erhalten, werden, wobei vorzugsweise das carboxylhältige Acylierungsmittel zu Cyclohexanon oder dem substituierten Cyclohexanon, gegebenenfalls in Kombination mit einem organischen Lösungsmittel, langsam zudosiert wird. Im Reaktionsgemisch anfallender Alkohol (Methanol, Ethanol, Propanol) kann über Kopf abdestilliert werden. Das molare Verhältnis zwischen Cyclohexanon oder substituiertem Cyclohexanon und dem carboxylhältigen Acylierungsmittel wird zwischen 100:1 und 0,5:1 gewählt. Bevorzugt beträgt das molare Verhältnis 20:1 und 1:1, besonders bevorzugt zwischen 10:1 und 1;5 zu 1. Als organische Lösungsmittel eignen sich besonders bevorzugt aliphatische oder aromatische, gegebenenfalls halogenierte Kohlenwasserstoffe wie etwa Benzol, Toluol, Xylole, Tetrachlorkohlenstoff u.s.w., aber auch Alkohole, wie Methanol, Ethanol, u.s.w.. Wird ein organisches Lösungsmittel zugesetzt, so beträgt das Volumsverhältnis zwischen organischem Lösungsmittel und Cyclohexanon oder dem substituierten Cyclohexanon zwischen 100:1 und 0,2 :1, bevorzugt zwischen 50:1 und 0,5:1 und besonders bevorzugt zwischen 10:1 und 1:1.
Als Säuren eignen sich sowohl anorganische als auch organische Säuren, die in äquimolarer Menge oder im molaren Über- oder Unterschuss bezogen auf das carboxylhältige Acylierungsmittel eingesetzt werden können. Insbesondere bevorzugt werden Salzsäure, Schwefelsäure, Phosphorsäure, p-Toluolsulfonsäure, Methansulfonsäure, u.s.w. Besonders bevorzugt wird ein stark saurer ionentauscher verwendet. Die Temperatur der Reaktionsmischung beträgt 50 bis 180 °C, bevorzugt 80 bis 150 °C und besonders bevorzugt 120 bis 135 °C. Das Ende der Reaktion wird durch eine geeignete Inprozeßkontrolle auf das Abreagieren des carboxylhältigen Acylierungsmittels festgestellt.
Nach erfolgter Reaktion wird überschüssiges Cyclohexanon oder überschüssiges substituiertes Cyclohexanon und bei Bedarf auch das organische Lösungsmittel unter vermindertem Druck aus dem Reaktionsgemisch entfernt. Das Gemisch der Reaktionsprodukte B und C kann gegebenenfalls unter Vakuum destilliert werden, wobei bevorzugt bei einem Vakuum < 500 mbar destilliert wird.

Das erfindungsgemäße Verfahren ist weiters dadurch gekennzeichnet, dass entweder das Reaktionsprodukt A oder das Gemisch der Reaktionsprodukte B und C entweder als Rohprodukt oder isoliert, gegebenenfalls in Kombination mit Methanol, Ethanol oder Diethylether, mit Hilfe eines Trägergases verdampft und katalytisch dehydriert wird, wobei als Dehydrierungskatalysatoren, jene, die dem Stand der Technik zur Aromatisierung von cyclischen Kohlenwasserstoffen entsprechen, verwendet werden, die beispielsweise aus J. Wiley Interscience, New-York, 1985; Houben-Weyl, Phenole, Vol.2, Georg Thieme- Stuttgart, 1976, 701-716 und J. H. Sinfelt in J. R. Anderson, M. Boudart: Catalysis Science and Technology, Vol. 1, Springer-Verlag, New York 1981 u.s.w.bekannt sind.
Wird Methanol, Ethanol oder Diethylether zugesetzt, so kann das molare Verhältnis zwischen dem Reaktionsprodukt A oder dem Gemisch der Reaktionsprodukte B und C und Methanol, Ethanol oder Diethylether zwischen 1:0,2 bis 100:0,2 gewählt werden, wobei ein molares Verhältnis zwischen 1:1 und 20:1 bevorzugt wird. Besonders bevorzugt ist ein molares Verhältnis zwischen 2:1 und 10:1.
Die katalytische Gasphasendehydrierung erfolgt bei einer Temperatur größer als 140 °C, bevorzugt bei einer Temperatur zwischen 200 und 300 °C und besonders bevorzugt bei einer Temperatur zwischen 220 und 270 °C. Als Katalysator wird bevorzugt Palladium oder Platin auf einem festen Träger, wie zum Beispiel Aluminium-oxid, der bevorzugt eine spezifische Oberfläche von mehr als 1 m²/g hat, in einer Konzentration zwischen 0.5 und 6 % verwendet.
Nach erfolgter Dehydrierung wird das in Kühlfallen gesammelte Kondensat destilliert, um 2-Coumaron oder substituierte 2-Coumarone in hohen Ausbeuten in reiner Form zu isolieren.

Das erfindungsgemäße Verfahren eignet sich somit zur Herstellung von Reaktionsprodukten aus Cyclohexanon oder substituierten Cyclohexanonen und einem carboxylhältigen Acylierungsmittel in hohen Ausbeuten von über 90 %, die anschließend in der Dampfphase katalytisch zu 2-Coumaron oder substituierten 2-Coumaronen in hohen Ausbeuten von über 70 % dehydriert werden.
Im Vergleich zum Stand der Technik werden mit dem erfindungsgemäßen Verfahren sehr einfach aus billigen Rohstoffen in nur einem einzigen Syntheseschritt flüssige, stabile Zwischenprodukte erhalten (Reaktionsprodukte A, B und C), die direkt durch katalytische Gasphasen-Dehydrierung zu 2-Coumaron oder substituierten 2-Coumaronen umgesetzt werden können.

### Beispiel 1:

196 g (2 mol) Cyclohexanon wurden in einem Doppelmantelreaktor vorgelegt und auf 125 °C erwärmt. Über einen Zeitraum von 60 Minuten wurden 120 g (1 mol) Glyoxylsäuremethylester-methyl-hemiacetal eingeleitet und entstehendes Methanol durch einen Stickstoffstrom aus dem Reaktionsgemisch ausgetragen. Nach weiteren 30 Minuten Reaktionszeit wurde bei 105 °C überschüssiges Cyclohexanon und geringe Mengen an Methanol unter reduziertem Druck aus dem Reaktionsgemisch entfernt. Durch nahezu quantitative Umsetzung erhielt man das Reaktionsprodukt A in 98 %i-ger Ausbeute mit einem Gehalt von 98 % (GC + HPLC).

### Beispiel 2:

62 g (0,33 mol) Reaktionsprodukt A wurden mit 300 ml Toluol und 6 g lonenfauscher Amberlyst 15 (Aldrich) versetzt. Nach 60 Minuten wurde die azeotrope Destillation beendet und der lonentauscher abfiltriert. Überschüssiges Toluol wurde unter vermindertem Druck vom Reaktionsgemisch beseitigt, und bei Bedarf wurde das Reaktionsgemisch destilliert (Siedepunkt: 90 °C bei 1 mbar).
Durch quantitative Umsetzung erhielt man ein Gemisch der Reaktionsprodukte B (cis-Form) und C, wobei sich das Gemisch laut HPLC Chromatogramm aus 95 % Reaktionsprodukt B und 4 % Reaktionsprodukt C zusammensetzte.

### Beispiel 3:

196 g (2 mol) Cyclohexanon und 30 ml konzentrierte Salzsäure wurden in einem Doppelmantelreaktor vorgelegt und auf 100 °C erwärmt. Über einen Zeitraum von 60 Minuten wurden 120 g (1 mol Glyoxylsäuremethylester-methyl-hemiacetal eingeleitet, und anschließend die Reaktionsmischung noch 30 Minuten bei 100 °C gekocht. Überschüssiges Cyclohexanon, Methanol, Wasser und Salzsäure wurden unter reduziertem Druck abdestilliert. Das Gemisch der Reaktionsprodukte setzte sich laut HPLC aus 68 % Reaktionsprodukt C, 28 % Reaktionsprodukt B (cis-Form) und 3 % cis-2-Oxocyclohexyliden-essigsäure zusammen. Das Reaktionsgemisch wurde durch Destillation bei einem Siedepunkt von 90 °C und einem Druck von 1 mbar destilliert. Ausbeute: 94 % des Gemisches der Reaktionsprodukte B und C bezogen auf Glyoxylsäuremethylester-methyl-hemiacetal.

### Beispiel 4:

56.1 g (0.5 mol) 4-Methyl-cyclohexanon und 18 g eines Gemisches aus Glyoxylsäuremethylesters und Glyoxylsäuremethylester-methyl-hemiacetal (ca. 80/20 nach Destillation) wurden in einem Doppelmantelreaktor vorgelegt und kontinuierlich auf 135 °C erwärmt. Der entstehende Methanol wurde unter Normaldruck aus dem Reaktionsgemisch ausgetragen. Nach weiteren 30 Minuten Reaktionszeit wurde bei 100 °C überschüssiges 4-Methyl-cyclohexanon und geringe Mengen an Methanol unter reduziertem Druck aus dem Reaktionsgemisch entfernt.
Durch nahezu quantitative Umsetzung erhielt man 2-(2-Oxo-5-methyl-cyclohexyl)-2-hydroxy-essigsäuremethyl-ester in hoher Ausbeute mit einem Gehalt von 95 % (GC).

### Beispiel 5:

128.5 g (1 mol) 4-Methoxy-cyclohexanon und 29 g (0.333 mol) Glyoxylsäuremethylester (frisch destilliert) wurden in einem Doppelmantelreaktor vorgelegt und kontinuierlich auf 135 °C erwärmt. Nach einer Reaktionszeit von 1.5 Stunden wurde bei 130 °C überschüssiges 4-Methoxy-cyclohexanon und geringe Mengen an Methanol unter reduziertem Druck aus dem Reaktionsgemisch entfernt.
Durch nahezu quantitative Umsetzung erhielt man 2-(2-Oxo-5-methoxy-cyclohexyl)-2-hydroxy-essigsäuremethyl-ester in 95 %iger Ausbeute mit einem Gehalt von 92 % (GC).

### Beispiel 6:

Die Produkte aus den Beispielen 1 bis 3 wurden jeweils mit Methanol im molaren Verhältnis 4 zu 1 verdünnt (das Gemisch der Reaktionsprodukte aus Beispiel 2 auch ohne Methanol) und in einem Verdampfer, der je nach Produkt auf einer Temperatur zwischen 210 bis 270 °C gehalten wurde, bei einer Flussrate von 15 ml/h Stickstoffstrom verdampft. Dieser Dampfstrom wurde in den Reaktorstickstoffstrom mit einer Flussrate von 200 bis 300 ml/h geleitet. Der mit 1 % Pd/Al₂O₃ Katalysator gefüllte, vertikal angeordnete Rohreaktor wurde bei einer Temperatur von 260 °C betrieben. Am Ende des Reaktors wurde der Gasstrom durch mehrere Kühlfallen geleitet und das Rohprodukt in einer mit Acetonitril gefüllten Falle kondensiert. Die Acetonitrillösung wurde anschließend mittels GC und HPLC analysiert. Bezogen auf die eingesetzte Menge an Edukt aus den Beispielen 1 bis 3 wurden Ausbeuten größer 70 % erzielt. Durch Erhöhung des Gasstromes konnten sogar Ausbeuten von ca. 80 % erzielt werden. Bei Bedarf wurde 2-Coumaron oder die substituierten 2-Coumarone unter reduziertem Druck destilliert, wobei der Gehalt an 2-Coumaron oder substituierten 2-Coumaronen 99.8% betrug (GC + HPLC).

## Patentansprüche

1. Verfahren zur Herstellung von 2-Coumaron oder substituierten 2-Coumaronen, **dadurch gekennzeichnet, dass** Cyclohexanon oder substituierte Cyclohexanone der Formel I 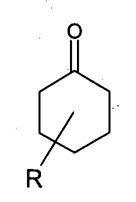 in der R H oder ein C₁-C₃-Alkyl-oder Alkoxyrest ist,
mit einem carboxylhältigen Acylierungsmittel der allgemeinen Formel IIa oder IIb 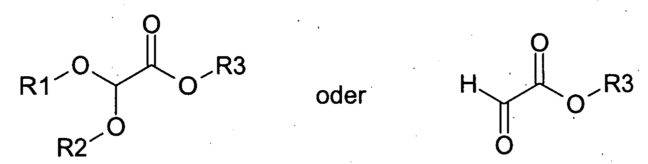 wobei R1, R2 und R3 unabhängig voneinander einen Wasserstoff oder einen Methyl-, Ethyl- oder Propyl-Rest bedeuten, mit der Maßgabe, dass R3 nur im Falle der Herstellung.von substituierten 2-Coumaronen H sein kann,
a) zu 2-(2-Oxo-cyclohexyl)-2-hydroxy-essigsäurealkylester oder substituierten 2-(2-Oxo-cyclohexyl)-2-hydroxy-essigsäurealkylestern umgesetzt werden, die
a₁) entweder direkt durch katalytische Gasphasen-Dehydrierung in 2-Coumaron oder substituierte 2-Coumarone überführt werden, oder
a₂) durch azeotrope Destillation unter basischen Bedingungen oder unter Verwendung einer starken Säure oder einem stark sauren lonentauscher zu einem Gemisch aus 2-Oxocyclohexyliden-essigsäurealkylester und dem Enol-Lacton der 2-Oxo-cyclohexyliden-essigsäure oder einem Gemisch aus substituiertem 2-Oxocyclohexyliden-essigsäurealkylester und dem Enol-Lacton der substituierten 2-Oxocyclohexyliden-essigsäure dehydratisiert wird, das anschließend wiederum durch katalytische Gasphasen-Dehydrierung in 2-Coumaron oder substituierte 2-Coumarone überführt wird, oder
b) direkt unter sauren oder basischen Bedingungen zu einem Gemisch aus 2-Oxocyclohexyliden-essigsäurealkylester und dem Enol-Lacton der 2-Oxocyclohexylidenessigsäure oder einem Gemisch aus substituiertem 2-Oxocyclohexyliden-essigsäurealkylester und dem Enol-Lacton der substituierten 2-Oxo-cyclohexyliden-essigsäure umgesetzt wird, das anschließend wiederum durch katalytische Gasphasen-Dehydrierung in 2-Coumaron oder substituierte 2-Coumarone überführt wird.

2. Verfahren nach Anspruch 1, **dadurch gekennzeichnet, dass** in Schritt a) oder b) das molare Verhältnis zwischen Cyclohexanon oder substituierten Cyclohexanonen und dem carboxylhältigen Acylierungsmittel zwischen 100 :1 und 0,5 :1 liegt.

3. Verfahren nach Anspruch 1, **dadurch gekennzeichnet, dass** die Reaktionstemperatur in Schritt a) oder b) über 50 °C liegt.

4. Verfahren nach Anspruch 1, **dadurch gekennzeichnet, dass** sich in Schritt a₂) oder b) als Säuren anorganische oder organische Säuren oder ein saurer Ionentauscher eingesetzt werden.

5. Verfahren nach Anspruch 4, **dadurch gekennzeichnet, dass** als Säuren p-Toluolsulfonsäure, Methansulfonsäure, Salzsäure, Schwefelsäure oder ein stark saurer lonentauscher verwendet werden.

6. Verfahren nach Anspruch 1, **dadurch gekennzeichnet, dass** in Schritt a₂) zur azeotropen Destillation und gegebenenfalls in Schritt b) zum Verdünnen der Reaktionsmischung während der Reaktion, aliphatische oder aromatische, gegebenenfalls halogenierte organische Lösungsmittel eingesetzt werden.

7. Verfahren nach Anspruch 1, **dadurch gekennzeichnet, dass** für die Gasphasendehydrierung in Schritt a₁), a₂) und b) sowohl die entsprechenden rohen Zwischenprodukte, als auch gereinigte Zwischenprodukte eingesetzt werden können.

8. Verfahren nach Anspruch 1, **dadurch gekennzeichnet, dass** die Dampfphasen-Dehydrierung bei einer Temperatur über 140°C ausgeführt wird.

9. Verfahren nach Anspruch 1, **dadurch gekennzeichnet, dass** bei der Gasphasen-Dehydrierung ein Katalysator bestehend aus Palladium oder Platin, der auf einem inerten, festen Träger mit einer spezifischen Oberfläche von mehr als 1 m²/g, aufgebracht ist, eingesetzt wird.

10. Verfahren zur Herstellung von 2-(2-Oxo-cyclohexyl)-2-hydroxyessigsäurealkylester oder substituierten 2-(2-Oxo-cyclohexyl)-2-hydroxyessigsäurealkyl-estern, **dadurch gekennzeichnet, dass** Cyclohexanon oder substituierte Cyclohexanone mit dem carboxylhältigem Acylierungsmittel umgesetzt werden, worauf der 2-(2-Oxo-cyclohexyl)-2-hydroxyessigsäurealkyl-ester oder substituierte 2-(2-Oxocyclohexyl)-2-hydroxyessigsäurealkyl-ester gegebenenfalls gemäß Anspruch 1, a₁) oder a₂) zu 2-Coumaron oder substituiertem 2-Coumaron weiterverarbeitet wird.
